# EUROPEAN PATENT APPLICATION

(11) **EP 4 664 466 A1**
(43) Date of publication of application: **17.12.2025**
(21) Application number: 25172014.0
(22) Date of filing: 23.04.2025
(51) Int. Cl.: G16H 10/20, G06F 21/60, G06N 3/02, G16H 10/65, G16H 40/63, G16H 50/70, G16H 80/00, H04L 12/54, G06F 11/00, G06F 11/07, H04L 1/00

(54) **DATA REPLICATION ARCHITECTURE USING MACHINE LEARNING FOR CLINICAL DATA FROM DIGITAL THERAPEUTIC APPLICATIONS**

(30) Priority: 11.06.2024 US 202418740338
(71) Applicant: Click Therapeutics, Inc., New York, NY 10013 (US)
(72) Inventor: Guttikonda, Sudheer, New York, 10013 (US); Tararache, Oana, New York, 10013 (US)
(74) Representative: Marks & Clerk LLP

(57) **Abstract**

Provided herein are systems and methods for maintaining integrity of data during clinical trials of digital therapeutic applications. A computing system can receive a data element generated based on interactions by a user with a digital therapeutic application during a clinical trial. The computing system can send the data element to each data store of a plurality of data stores. The computing system can access a first data store to retrieve a first instance of the data element. The computing system can identify the first instance of the data element as invalid. The computing system can access a second data store to retrieve a second instance of the data element, responsive to the identifying the first instance of the data element in the first data store as invalid. The computing system can store the second instance of the data element onto a data repository for the clinical trial.

## Description

### CROSS-REFERENCE TO RELATED APPLICATIONS

The present applications claim priority to and the benefit of U.S. Non-Provisional Application. No. 18/740,338, titled "DATA REPLICATION ARCHITECTURE USING MACHINE LEARNING FOR CLINICAL DATA FROM DIGITAL THERAPEUTIC APPLICATIONS", and filed on June 11, 2024, which is incorporated by reference in its entirety.

### BACKGROUND

With increasing reliance on data by servers hosting resources for applications on user devices, data integrity may be more focal for such servers in properly allocating and selecting resources to provide to user devices. What is more, maintaining data integrity may be particularly paramount in certain types of applications. For example, with digital therapeutic applications, a server may aggregate data related to user interaction and performance and then rely on the data to select the proper digital therapeutic interventions for the user. These digital therapeutic interventions may be provided by the server in the form of messages to alleviate or treat user's various medical conditions. Furthermore, the data may be aggregated in the context of a clinical trial to assess the efficacy and performance of the digital therapeutic application and may thus be vital for properly making such assessments.

Even slight reductions in the integrity of the aggregated data may adversely impact the selection and delivery of proper digital therapeutic interventions. The diminution in data validity may also negatively affect the ability to make proper assessments of the digital therapeutic application in the context of clinical trials. Both of these may severely diminish the effectiveness of the therapy in addressing the user's conditions. From a hardware perspective, the reduction in data integrity may result in wasted consumption of computing and network resources (e.g., processing, memory, and network bandwidth) as well as a reduction in the performance and loss of functionality of the remote server and the user device. Furthermore, from a human-computer interaction (HCI) perspective, the reduction in performance and loss in functionality may lead to a reduction in the quality of HCI between the user and the digital therapeutics application.

Between the time of transmission from the user device and subsequent retrieval from the storage, the data may become unusable for a multitude number of reasons. For instance, an interruption in service in the storage or error on the storage disks themselves may result in a reduction in data integrity. This problem may become exacerbated, when a significant amount of user interaction data is accumulated on the user device while offline and then sent all at once shortly after establishing connection with the remote server. The reduction in data integrity may result in the remote server being unable to select the proper function or identify the appropriate resources to provide to the user device.

There may be a number of technical challenges with transferring and storing substantial amounts of data, especially when the user device transfers a large amount of data upon connecting with the server. In this case, the user device may accumulate large amounts of data, such that when the user device sends the data to the remote server upon establishing a connection, the likelihood of data integrity may be reduced. In the context of digital therapeutics, the reduction in data integrity may worsen issues with proper selection of digital therapeutic content and functionality. This may be especially so because interaction data may be sparse, especially for newer users of the application with insufficient time using the application, making it difficult to evaluate the performance or efficacy of the digital therapeutic content provided through the digital therapeutic application during a clinical trial.

Furthermore, the inability of accurately and precisely receiving data of user interactions may result in inefficient allocation of resources (e.g., processing, memory, and network bandwidth) for delivering subsequent data for presentation of content and interactivity through the user interface elements. The reduction of data integrity may lead to skewed or inaccurate clinical trial results, or worse inability to properly assess the efficacy and performance of the digital therapeutic application, thereby leading to ineffective digital therapeutics provided to the user and thus resulting in little to no effect on the condition of the user. This in turn may also result in wasted computing resources from providing ineffective digital therapeutics and lowering the quality of HCI between the user and the application.

### SUMMARY

The present disclosure relates to systems and methods for maintaining the integrity and reliability of data generated at user devices in clinical trials especially in the realm of digital therapeutics. Given the critical role data plays in achieving the primary endpoints of clinical trials, ensuring the integrity and reliability of data can reduce the costs of clinical trials as well as improve the accuracy of the results of the clinical trials, to make more certain that treatments developed are effective for patient use. In particular, the new data replication system disclosed herein can interface with a set of data stores to replicate data associated with a digital therapeutics application across each of the data stores and then reconcile the data from the data stores onto a data repository. These data stores may serve to store replicas of the data and the data repository may function as a centralized storage to store the data from each of the data stores. There may be a myriad of advantages and benefits from this approach. This architecture may mitigate any risk to data integrity, thereby bolstering the overall quality of the data used to evaluate the efficacy of digital therapeutics. The addition of machine learning techniques may also further enhance the quality of the data gathered from the data stores. Ultimately, this may lead to more accurate assessments of therapeutic outcomes and improves the allocation of digital therapeutic content, enhancing patient treatment effectiveness and conserving computing resources.

The issue with data integrity may be particularly problematic when the source for the data (e.g., the user device) transitions often between a connected and a disconnected state with respect to the server over the network. The digital therapeutic application disclosed herein may be uniquely built for frequent use in an offline state for convenience of the user. When the user device is in an offline state, the digital therapeutic application on the user device may accumulate or aggregate data generated from the user interacting with the therapeutic interventions provided through the user interface elements of the application. Depending on the type of data, the complexity of the processing operations, and the rate of user's interactions with the user interface elements, the amount of data from the digital therapeutics application may be substantial. When the user device establishes a connection with the server, the digital therapeutics application can send the user interaction data all at once to the remote server. Upon receipt, the remote server may store the data associated with the user interaction on storage for later retrieval and use, such as to perform analytics or identify content to provide to the user.

To address these and other challenges, a data replication system of the server can interface with a set of data stores to replicate data and with a central data repository to better ensure data integrity. The data stores may serve to store replicas of the data and the data repository may function as a central storage to aggregate the data from each of the data stores. To that end, the server may provide digital therapeutic content to the user device for presentation via the user interface. The digital therapeutic content may direct the user to perform an activity to address a targeted condition of the user. For example, the digital therapeutic content may be a prompt to perform an activity that may be in accordance with a particular task of the clinical trial, such as an emotional faces memory task (EFMT) or attention bias modification treatment (ABMT), among others.

In addition, the digital therapeutic application disclosed herein may be uniquely and particularly configured to operate on the user device for frequent use in an offline state. In particular, the digital therapeutic application may be designed and optimized to function without a constant connection with the server. For one, the digital therapeutic application may include core functionalities of providing digital therapeutic content to the user, without reliance on a constant connection with the server hosting resources. This may allow for a seamless experience for the user, independent of connectivity with the server. For another, the digital therapeutic application may store data locally on the user device, with functionality to write and store new data onto storage when not connected with the server. When the connection is re-established with the server, the digital therapeutic application may synchronize with the server, receiving any updates to the digital therapeutic content from the server and by providing data accumulated while offline to the server. This may allow for efficient management of computing resources on the user device locally by the digital therapeutic application.

During the session, the user device may monitor interaction during a session and may generate a set of data elements based on the interaction data. Each data element may include various information related to the interactions and contextual factors, such as the type of task, a location at which the user performed the task, a timestamp corresponding to the performance of the task, and an indication of whether the user response is correct with respect to the task. The digital therapeutic application on the user device may obtain a portion of the data elements when disconnected from the server providing the digital therapeutic application. When the user device becomes connected with the server, the digital therapeutic application may send the send data elements to the server.

Upon receipt of the data from the user device, the data replication system may communicate with the data stores to store instances of the one or more data elements within each data store. The instances may correspond to copies or replications of the interaction data generated on the digital therapeutic application. Upon receipt, each data store may store and maintain the instances of the data elements. The instance in one data store may differ from the instance in a subsequent data store. For example, each data store may store the instances of the data element in accordance with the respective data store schema, and as a result the instance of the data element in one data store may be different from another instance of the data element in a different data store.

At a subsequent time, the data replication system may access a data store to retrieve the instance of the data elements. The data replication system may first identify which data store to retrieve leveraging machine learning models. For each instance of a given data element, the data replication system may identify whether the instance is valid or invalid. An invalid instance may correspond to a distortion (e.g., in format or structure), unexpected value (e.g., outside a range for a given type of metric), reduction in data integrity (e.g., in the value of the data element itself), data inconsistencies (e.g., incongruence with another data element), or missing value in the data element (e.g., missing side effects, frequency of medication, or portions of a journal), among others. The invalid instances may lead to skewing or invalidation of clinical data for further analysis of the digital therapeutic application. To identify whether the data is valid or invalid, the data replication system may use a trained machine learning model to determine a confidence value to indicate a degree of validity based on the instance of the data element. For example, the confidence value for a data element with a missing value or a value outside the expected range may be less than the confidence value for a data element with a complete value or a value within the expected range.

If the confidence value satisfies (e.g., greater than or equal to) a threshold, the data replication system may identify the instance of the data element as valid and may store the instance of the data element on the data repository for the clinical trial. Otherwise, if the confidence value does not satisfy (e.g., less than) the threshold, the data replication system may identify the instance of the data element as invalid. In addition, the data replication system may access another data store to retrieve a different instance of the corresponding data element. The data replication system may repeat the identification of whether the data element from the other data store is valid or invalid. When the data element is identified as valid, the data replication system may store the instance of the data element onto the data repository. On the other hand, when the data element is identified as invalid, the data replication system may access yet another data store and repeat the process.

As a result of replicating the data elements and storing the data elements identified as valid, onto the data repository, the data replication system may improve the data integrity for the clinical trial, even when receiving data that is collected when the computing system is in an offline state. Furthermore, there are several technical advantages and benefits with the ability to replicate clinical trial results across a plurality of data stores. For one, the replication of the interaction data may provide multiple copies of the interactions within each data store to store instances of the data associated with the clinical trials, when the user device changes states from online to offline state. With each data store maintaining respective instances of interaction data to maintain a record of the data associated with the clinical trial, this may increase data integrity between each instance of data by ensuring that data elements with satisfactory confidence values are stored on the data repository.

The data on the data repository may be used to evaluate the performance and efficacy of the digital therapeutic application properly and more accurately, thereby compensating efforts and time exhausted in performing the clinical trial. The data on the data repository may be also used by the server to properly select and allocate digital therapeutic content with higher efficacy to provide to the user device. This may reduce consumption of computing resources (e.g., processor, memory, and network bandwidth) that would have otherwise been wasted in delivering ineffective digital therapeutics to the user. From the perspective of the user, the provision of higher performance and more effective digital therapeutics may lead to an improvement or amelioration of the condition of the user. Furthermore, the particular and unique configuration of the digital therapeutic application for use even when not connected with the server may greatly enhance the utility and functionality of the user device, thereby increasing the quality of HCI.

Aspects of the present disclosure are directed to systems and methods for maintaining integrity of data during clinical trials of digital therapeutic applications. One or more processors coupled with memory can receive a data element generated based on at least one interaction by a user with digital therapeutic application. The one or more processors can send the data element to each data store of a plurality of data stores. The one or more processors access a first data store of the plurality of data stores to retrieve a first instance of the data element. The one or more processors can access a second data store of the plurality of data stores to retrieve a second instance of the data element, responsive to determining the first instance of the data element in the first data store as invalid. The one or more processors can store the second instance of the data element onto a data repository.

In some embodiments, the one or more processors can determine that a first instance of a second data element on the second data store is invalid. The one or more processors can provide an indication that the second data element on the first data store and the second data store is invalid. The one or more processors can store the first instance of the data element.

In some embodiments, the one or more processors can store a second instance of the second data element from the first data store onto the data repository, without accessing the second data store to retrieve a third instance of the second data element. The one or more processors can determine a confidence value indicating a degree of validity of the first instance of the data element. The one or more processors can determine that the confidence value does not satisfy a threshold metric. In some embodiments, the one or more processors can apply a machine learning model to determine the confidence value. The one or more processors can determine a plurality of metrics corresponding to at least one of a degree of efficacy of the digital therapeutic application, a degree of performance of the digital therapeutic application, or a degree of severity of a condition to be addressed in the user, using the data element stored on the data repository.

In some embodiments, the one or more processors can provide a graphical user interface comprising information associated with at least one of the plurality of data stores or the data element on the data repository. In some embodiments, the one or more processors can determine the first data store of the plurality of data stores by applying a machine learning (ML) model. The one or more processors can retrieve from a user device executing the digital therapeutic application, a plurality of data elements generated over a time period during which no communications were established with the user device. The one or more processors can provide a respective instance of the data element to each data store of the plurality of data stores, the respective instance comprising an encrypted copy of the data element. The digital therapeutic application is configured to generate the data element, in at least partial concurrence with the user being on a medication to address a condition.

### BRIEF DESCRIPTION OF THE DRAWINGS

The foregoing and other objects, aspects, features, and advantages of the disclosure will become more apparent and better understood by referring to the following description taken in conjunction with the accompanying drawings, in which:
FIG. 1 depicts a block diagram of a system of maintaining integrity of data during clinical trial for digital therapeutics applications, in accordance with an illustrative embodiment;
FIG. 2 depicts a block diagram of a process of generating data elements based on interactions between a user and a digital therapeutics application, in accordance with an illustrative embodiment;
FIG. 3 depicts a block diagram of a process of accessing the plurality of data stores to retrieve an instance of a data element to validate the data element and store the data element in a data repository for the clinical trial, in accordance with an illustrative embodiment;
FIG. 4 depicts a block diagram of a process of generating a plurality of metrics using the data elements from the data repository and transmitting an output to an administrative computing device, in accordance with an illustrative embodiment;
FIG. 5 depicts an example of a dashboard provided by the data replication system, in accordance with an illustrative embodiment;
FIG. 6 depicts a block diagram of data replication architecture for clinical data, in accordance with an illustrative embodiment;
FIG. 7 depicts a flow diagram of a method of maintaining integrity of data during clinical trial for digital therapeutics applications, in accordance with an illustrative embodiment; and
FIG. 8 is a block diagram of a server system and a client computer system in accordance with an illustrative embodiment.

### DETAILED DESCRIPTION

For purposes of reading the description of the various embodiments below, the following enumeration of the sections of the specification and their respective contents may be helpful:
Section A describes systems and methods for data replication of data associated with clinical trials for digital therapeutics applications; and
Section B describes a network and computing environment which may be useful for practicing embodiments described herein.

### A. Systems and Methods for Data Replication of Data Associated with Clinical Trials for Digital Therapeutic Applications

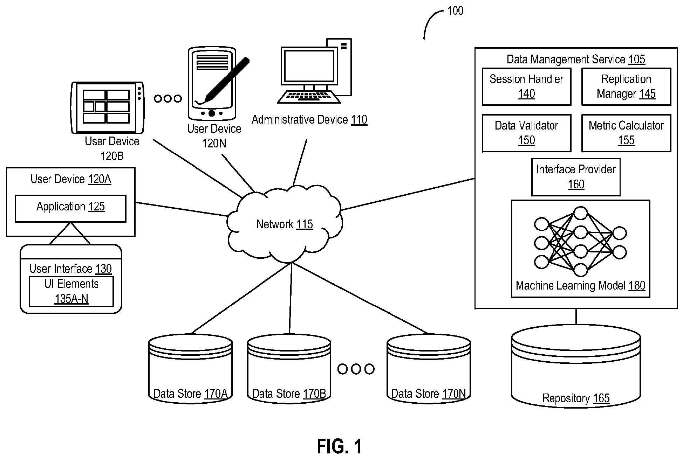

Referring now to FIG. 1, depicted is a block diagram of a system 100 of maintaining integrity of data during clinical trials for digital therapeutics applications. In an overview, the system 100 may include at least one data management service 105, an administrative device 110, a set of user devices 120A-N (hereinafter generally referred to as user devices 120), a plurality of data stores 170A-N (hereinafter generally referred to as data stores 170), and a repository 165, communicatively coupled with one another via at least one network 115. At least one of the user devices 120 (e.g., the first user device 120A as depicted) may include at least one application 125. The application 125 may include or provide at least one user interface 130 with one or more user interface (UI) elements 135A-N (hereinafter generally referred to as UI elements 135). The data management service 105 may include at least one session handler 140, at least one replication manager 145, at least one data validator 150, at least one metric calculator 155, at least one interface provider 160, and at least one machine learning (ML) model 180, among others. The data management service 105 may include or have access to at least one repository 165. The functionalities of the application 125 on the user device 120 may be performed in part on the data management service 105, and vice-versa. Each of the components of the system 100 can be implemented using the computing system as described in Section B.

In further detail, the data management service 105 (sometimes herein generally referred to as a messaging service) may be any computing device comprising one or more processors coupled with memory and software and capable of performing the various processes and tasks described herein. The data management service 105 may be associated with an entity administering provision of resources and content for the application 125 or delivery of the digital therapeutic to the user of the user device 120. In some embodiments, the data management service 105 may be associated with an entity overseeing at least one clinical trial associated with the digital therapeutic provided through the application 125. The data management service 105 may be in communication with the one or more user devices 120 and the repository 165 via the network 115. The data management service 105 may be situated, located, or otherwise associated with at least one computer system. The computer system may correspond to a data center, a branch office, or a site at which one or more computers corresponding to the data management service 105 are situated.

Within the data management service 105, the session handler 140 can manage a session and receive interactions from a user. The replication manager 145 can identify data elements to disperse within each data store 170. The data validator 150 can validate the data elements of each data store 170 and store the validated data element within the repository 165. The metric calculator 155 can calculate metrics associated with the data elements within the repository 165. The interface provider 160 can provide a dashboard of the metrics associated with the data elements. In some embodiments, the data management service 105 may be part of a service (e.g., with the functionalities of the session handler 140 as depicted) managing the session. In some embodiments, the data management service 105 may be separate from the service. For instance, the data management service 105 may include the functionalities of the replication manager 145, the data validator 150, and the metric calculator 155, and may be in communication with the separate service (e.g., with the functionalities of the session handler 140 as depicted) to exchange interaction data through the service.

The ML model 180 may include any machine learning model or artificial intelligence (AI) algorithm to select data stores 170 and determine whether instances of data elements are valid. In some embodiments, the data management service 105 may have one ML model 180 to select data stores 170 and another ML model 180 to determine whether data elements are valid. The architecture for the machine learning model of the prediction model 180 can include, for example, a deep learning neural network (e.g., convolutional neural model architecture), a regression model (e.g., linear or logistic regression model), a random forest, a regression model (e.g., linear or logistic), a support vector machine (SVM), a clustering algorithm (e.g., k-nearest neighbors), or a Naive Bayesian model, among others. In general, the prediction model 180 may have at least one input and one output. The input and output may be related via a set of weights. The input may include various data about the data stores 170 and data elements stored thereon. The output may include a selection indication and confidence value. The set of weights can be in accordance with the machine learning architecture. The ML model 180 can be trained using the training data (e.g., in accordance with supervised learning). In general, the ML model 180 may include a set of inputs and a set of outputs, related to one another via a set of weights. The weights may be arranged in accordance with the architecture for the machine learning model.

The administrative device 110 may be any computing device comprising one or more processors coupled with memory and software and capable of performing the various processes and tasks described herein. The administrative device 110 may be in communication with the data management service 105 and the data stores 170 via the network 115. The administrative device 110 may be a laptop computer, a tablet computer, or a smart phone to receive an output of the data management service 105. The output may trigger one or more UI elements of a user interface of the administrative device 110 to provide a dashboard for managing data associated with the clinical trial.

The user device 120 (sometimes herein referred to as an end user computing device) may be any computing device comprising one or more processors coupled with memory and software and capable of performing the various processes and tasks described herein. The user device 120 may be in communication with the data management service 105 and the data stores 170 via the network 115. The user device 120 may be a smartphone, other mobile phone, tablet computer, wearable computing device (e.g., smart watch, eyeglasses), or laptop computer. The user device 120 may be used to access the application 125. In some embodiments, the application 125 may be downloaded and installed on the user device 120 (e.g., as a native or mobile app from a digital distribution platform). In some embodiments, the application 125 may be a web application with resources accessible via the network 115. The functionality of the application 125 may be independent of connections with the network 115.

The application 125 executing on the user device 120 may be a digital therapeutics application. The application 125 may present or provide a session (sometimes referred to herein as a therapy session) in accordance with a clinical trial to address at least one condition in the user. The condition of the end user may include, for example, chronic pain (e.g., associated with or including arthritis, migraine, fibromyalgia, back pain, Lyme disease, endometriosis, repetitive stress injuries, irritable bowel syndrome, inflammatory bowel disease, and cancer pain), a skin pathology (e.g., atopic dermatitis, psoriasis, dermatillomania, and eczema), a cognitive impairment (e.g., mild cognitive impairment (MCI), Alzheimer's, multiple sclerosis, and schizophrenia), a mental condition (e.g., an affective disorder, bipolar disorder, obsessive-compulsive disorder, borderline personality disorder, and attention deficit/hyperactivity disorder), a substance use disorder (e.g., opioid use disorder, alcohol use disorder, tobacco use disorder, or hallucinogen disorder), and other conditions (e.g., narcolepsy and oncology or cancer), among others.

The end user may be on medication to address the condition, in at least partial concurrence with the use of the application 125 (e.g., for any number of sessions). For instance, if the medication is for pain, the end user may be taking acetaminophen, a nonsteroidal antiinflammatory composition, an antidepressant, an anticonvulsant, or other composition, among others. For skin pathologies, the end user may be taking a steroid, antihistamine, or topic antiseptic, among others. For cognitive impairments, the end user may be taking cholinesterase inhibitors or memantine, among others. For a mental condition, the end user may be taking antidepressants, mood stabilizers, antipsychotics, anxiolytics, or stimulants, among others. For substance abuse disorders, the end user may be taking a naltrexone, disulfiram, acamprosate, or nicotine replacement therapy, among others. The end user may also participate in other psychotherapies for these conditions. In some embodiments, the digital therapeutic content may be provided to the end user within the digital therapeutics application towards achieving an endpoint of the end user. An endpoint can be, for example, a physical or behavioral goal of an end user, a completion of a medication regimen, or an endpoint indicated by a doctor or an end user. At least one of the user devices 120 may have a digital therapeutics application and may provide a session (sometimes referred to herein as a therapy session) to address at least one condition of the end user. The application 125 may increase efficacy of the medication that the user is taking to address the condition.

The application 125 can include, present, or otherwise provide at least one user interface 130 including the one or more user interface elements 135A-N (hereinafter generally referred to as UI elements 135) to a user of the user device 120. The user interface 130 may be provided in accordance with a configuration on the application 125. The UI elements 135 may correspond to visual components of the user interface 130, such as a command button, a text box, a check box, a radio button, a menu item, and a slider, among others. In some embodiments, the application 125 may be a digital therapeutics application and may provide a session (sometimes referred to herein as a therapy session) via the user interface 130 to address the condition. The user interface 130 may include the set of UI elements 135 to present digital therapeutic content.

The digital therapeutic content may be in any modality, such as text, image, audio, video, or multimedia content, among others, or any combination thereof. The content items can be stored and maintained in the repository 165 using one or more files. For instance, for text, the digital therapeutic content can be stored as text files (TXT), rich text files (RTF), extensible markup language (XML), and hypertext markup language (HTML), among others. For an image, the digital therapeutic content may be stored as a joint photographic experts' group (JPEG) format, a portable network graphics (PNG) format, a graphics interchange format (GIF), or scalable vector graphics (SVG) format, among others. For audio, the digital therapeutic content can be stored as a waveform audio file (WAV), motion pictures expert group formats (e.g., MP3 and MP4), and Ogg Vorbis (OGG) format, among others. For video, the digital therapeutic content can be stored as a motion pictures expert group formats (e.g., MP3 and MP4), QuickTime movie (MOV), and Windows Movie Video (WMV), among others. For multimedia content, the digital therapeutic content can be an audio video interleave (AVI), motion pictures expert group formats (e.g., MP3 and MP4), QuickTime movie (MOV), and Windows Movie Video (WMV), among others.

The digital therapeutic content may include a set of stimuli (e.g., in the form of audio, visual, or text) for the user to conduct a particular task in accordance with the clinical trial. The task may include, for example, an implicit association task (IAT) (e.g., associating stimuli with concepts); an attention bias modification training (ABMT) (e.g., training users to shift attention away from certain stimuli); an emotional faces memory task (EFMT) (e.g., testing users to recognize and remember certain facial emotions); digital support tool (DST) (e.g., providing messages based on a state of user); and adaptive goal setting (AGS) (e.g., providing messages based on dynamic objectives for user); among others.

The application 125 may be provided to the user device 120 as part of a clinical trial. The clinical trial may be administered, supervised, or otherwise carried out by the entity associated with the data management service 105. The clinical trial may include a study or investigation to evaluate a performance or efficacy of the digital therapeutics provided through the application 125 to the user of the user device 120. The clinical trial may be of a set duration, ranging between 7 days to 6 months. The clinical trial may be defined in terms of an objective, a study design, a cohort, a type of intervention, a control, and one or more outcome measures, among others. The objective may include assessment of the impact or effect of the digital therapeutic to the user through the application 125. The design may include, for example, an exploratory trial, basket study, a pragmatic trial, a comparative study, a randomized control trial (RCT), or a single-arm study, among others. The cohort (or study population) may identify which subset population is eligible to participate in the clinical trial. The intervention may identify which type of tasks the users are to be directed to perform via the application 125. The control may identify whether a subset of users are to receive a placebo or the digital therapeutic intervention. The outcome measures may define one or more metrics associated with the condition, such as symptom severity, health outcome, adherence rate, biomarkers, or behavioral data, among others.

The repository 165 may be a centralized data storage to store and maintain data from the set of data stores 170. The data from the set of data stores 170 can be structured, semi-structured, or unstructured. In some embodiments, the repository 165 may be a data lake to collect, store, or otherwise manage clinical trial related data. The repository 165 may collect structured clinical trial data (e.g., patient demographics, medical records, or trial protocol), semi-structured, and unstructured data (e.g., physician notes, lab results, imaging scans, or patient-reported outcomes), among others. The repository 165 can prevent reduction in the data integrity for patient information during clinical trial collection. The repository 165 may include metadata to organize, sort, or otherwise catalog the clinical trial data. For example, the metadata may organize the clinical trial data such that a user (e.g., a clinician or administrator of the clinical trial) can navigate the repository 165 to the relevant clinical trial data.

In some embodiments, the repository 165 may be a structured database (e.g., relational database management systems (DBMS)) to store clinical trial data in a structured format. The format can be structured based on a protocol to define at least one of data types, data relationships, data constraints, among others. Using the tables, a user (e.g., a clinician) may access the data using a structure query language (SQL). While running various operations, the data management service 105 and the application 125 may access the repository 165 to retrieve identified data therefrom. The data management service 105 and the application 125 may write data onto the repository 165 from running such operations.

Each data store 170 may store and maintain various resources and data associated with the data management service 105 and the application 125. Each data store 170 can be a repository that is structured, unstructured, or semi-structured. Each data store 170 can include cloud storage services, file systems, data lakes, key-value stores, and the like. In some embodiments, the data stores 170 may include a database management system (DBMS) to arrange and organize the data maintained thereon, such as instances of data elements, among others. The DBMS may include a structured collection of data using tables with organized schemas. For instance, the DBMS can be a SQL database or a NoSQL database. In some embodiments, the data store 170 may include an event queue (or a message queue) to store and maintain data associated with events (e.g., on the user device 120 or elsewhere). The data stores 170 may be in communication with the data management service 105 and the one or more user devices 120 via the network 115. While running various operations, the data management service 105 and the application 125 may access the data store 170 to transmit and retrieve identified data therefrom. The data management service 105 and the application 125 may also write data onto the data stores 170 from running such operations.

In some embodiments, at least one data store 170 may be associated with a third-party entity. In some embodiments, the third-party entity can be at least one of a pharmaceutical company, laboratories, health information exchange networks, health insurance companies, medical research institutions, medical imaging centers, medical education institutions, or hospitals, among others. For example, the data management service 105 may access the data store 170A of a hospital to store the clinical trial data associated with a patient. In some embodiments, at least one data store 170 may be associated with the entity supervising the clinical trial (e.g., the same entity as the data management service 105).

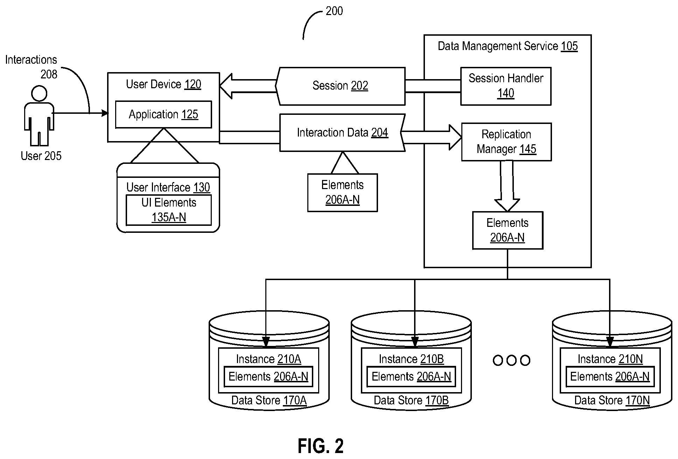

Referring now to FIG. 2, depicted is a block diagram of a process 200 of generating data elements based on interactions between a user and a digital therapeutics application to replicate the data elements. The process 200 may include or correspond to operations performed by the system 100 to generate data elements from an interaction with a session. Under the process 200, the session handler 140 executing on the data management service 105 may send, transmit, or otherwise provide instructions for at least one session 202 to be presented via the application 125. The instructions may identify or include the clinical trial content to be presented via the user interface 130 for the application 125 to a user 205. The instructions may include, for example, a specification as to which UI elements 135 are to be used and may identify content associated with the clinical trial to be displayed on the UI elements 135 of the user interface 130. The instructions may be code, data packets, or a control to present the session to the user 205 via the application 125 running on the user device 120.

For the session 202, the session handler 140 may create, write, or otherwise generate the instruction. The generation of the instruction may be based on the digital therapeutic content associated with the user 205. For example, the session handler 140 may select the contents of the application 125 based on a digital therapeutic content associated with the user 205. In some embodiments, the session handler 140 may generate the instructions for the session 202 to include digital therapeutic content, including messages to the user 205. For instance, the messages may include exclusion criteria for the clinical trial. In some embodiments, the session handler 140 may generate the instruction for the session 202 to include digital therapeutic content to prompt the user 205 to perform a certain task. The clinical trial may include an objective, inclusion criteria, study design, exclusion criteria, intervention outcome measures, study duration, statistical analysis, expected benefits, potential risk, and ethical considerations, among others. The session 202 may correspond to a set number of tasks to be performed by the user 205 within a defined time period. With the generation, the session handler 140 may transmit the instruction for the session 202 to the user device 120.

The application 125 on the user device 120 may retrieve, identify, or otherwise receive the instructions for the session 202. The application 125 may perform, conduct, or otherwise execute the instructions for the session 202. Based on the instructions, the application 125 may render, display, or otherwise present the digital therapeutic content via the UI elements 135 of the user interface 130. For example, the user 205 may participate in a clinical trial assessing the efficacy of performing a task to address mild cognitive impairment (MCI). The session 202 may include messages for how often activities associated with the task are to be performed during the course of the clinical trial. In some embodiments, the application 125 may include the instructions for the session 202 (e.g., pre-loaded or installed). In some embodiments, the application 125 may execute the session 202 subsequent to receipt (e.g., independent of connection with the network 115).

Upon the presentation of the content for the session 202, the application 125 on the user device 120 may detect, record, or monitor at least one interaction 208 by the user 205 with the application 125. Each interaction 208 may correspond to one or more tasks, actions, interventions, assessments, or criteria, among others. The application 125 may use event listeners or handlers on the UI elements 135 of the user interface 130 to monitor for interactions 208. In some embodiments, the application 125 may use listeners associated with input/output devices on the user device 120 to monitor for interactions 208. For instance, the application 125 may use actionable objects on the user interface 130 to detect button presses, fingerprints, and keyboard interactions, among others.

Based on the interactions 208, the application 125 may write, create, or otherwise generate interaction data 204 identifying the interactions 208 by the user 205 with the session 202 for the clinical trial. In some embodiments, the application 125 may generate the interaction data 204 identifying interactions 208 with the clinical trial over an amount of time (e.g., 1-10 interactions 208 with the session 202 provided to the user 205). The user device 120 may be offline (e.g., disconnected from Wi-Fi, Cellular Data, internet providers, among others) for an amount of time (e.g., one minute, one hour, one day, one week, one month, among others). When the user device 120 is offline, the data management service 105 may be unable to establish a communication with the user device 120. For instance, the user device 120 may be offline for 1-3 days, however, the application 125 may register the interactions 208 of the user 205. In some embodiments, the interaction data 204 may include a log of interactions 208 detected on the UI elements 135 of the user interface 130 presenting the digital therapeutic content and information derived from a set of interactions 208. The interaction data 204 may include timestamps and metadata to reference individual interactions with the session 202, digital therapeutic content of the session 202, or the application 125 of the user device 120, among others.

The timestamp may identify, for example, a time at which the digital therapeutic content of the session 202 is presented to the user 205, a time at which the session 202 is provided to the user 205, a time at which the user device 120 is reported to be offline, a duration in which the user device 120 is offline, and a time at which the user 205 interacts with the user interface 130 of the application 125, among others. In some embodiments, the interaction data 204 may include an identifier to distinguish between which sessions 202 the user 205 interacts with. For instance, the user 205 may be involved in one or more clinical trials, thereby, the session handler 140 may provide one or more sessions 202 to the user device 120. When the user 205 interacts with each session 202, the interaction data 204 may include an identifier to correspond to each session 202.

The interaction data 204 can include a set of data elements 206A-N (hereinafter generally referred to as elements 206). Each of the data elements 206 may include information associated with the interactions 208. In some embodiments, the data element 206 may be structured and may include one or more fields and corresponding one or more values. Each field may include various measures, such as a type of interaction, a timestamp associated with the interaction, an indication of whether the interaction is a correct or incorrect response for the task, user trait data, medical history, baseline characteristics, indication of adverse events (i.e., side effects), efficacy outcomes, safety assessment, adherence data, participant reported outcomes, quality of life measures, or data on concomitant medications, among others. For instance, the user 205 may report side effects, compliance data, and efficacy outcomes of the digital therapeutic content within the actionable objects of the application 125. The application 125 may generate interaction data 204, including the side effects, compliance data, and the efficacy outcomes as the elements 206, among others. In some embodiments, the data element 206 may be unstructured, and may include similar or same information described above with respect to the structured data. For example, the data element 206 may include free text data identifying patient-reported outcomes inputted by the user 205.

The user device 120 may be offline or otherwise disconnected from the data management service 105 for a certain amount of time (e.g., 1 hour to 4 days). During this time, the application 125 may continue to generate interaction data 204 and store and maintain the interaction data 204 locally. For instance, the application 125 may generate a first interaction data 204, a second interaction data 204, and a third interaction data 204 when the user device 120 is offline. Each interaction data 204 can have corresponding elements 206 (e.g., first interaction data 204 and first elements 206, second interaction data 204 and second elements 206). Once the device is online, the user device 120 may establish, link, or otherwise begin communications with the data management service 105. In response to the establishment of the connection, the application 125 can send, provide, or otherwise transmit the interaction data 204 (e.g., first interaction data 204, second interaction data 204, and third interaction data 204) to the data management service 105.

The replication manager 145 can receive, identify, or otherwise retrieve the interaction data 204 from the user device 120. The replication manager 145 may retrieve the interaction data 204 when the user device 120 establishes communication with the data management service 105. In some embodiments, the application 125 may transmit, send, or otherwise provide a signal to the data management service 105 to identify that the user device 120 has transitioned to an online state. The online state can indicate that the device is connected to a wireless or wired internet connection. For instance, prior to the application 125 transmitting the interaction data 204 to the replication manager 145 of the data management service 105, the application 125 may transmit the signal to indicate that the user device 120 is in an online state and ready to synchronize with the data management service 105. Upon reception of the signal, the data management service 105 may invoke the replication manager 145 by transmitting a preamble of the signal to the replication manager 145.

With the reception of the interaction data 204, the replication manager 145 may parse or process the interaction data 204 to extract, obtain, or otherwise identify the elements 206 from the interaction data 204. In some embodiments, the replication manager 145 may generate, create, or otherwise determine the elements 206 using the interaction data 204. In some embodiments, the signal from the application 125 may include labeling, mappings, or associations for the replication manager 145 to identify the elements 206. For instance, the actionable objects involved with the interactions 208 may include data fields which indicate the elements 206 for the replication manager 145. In some embodiments, to extract the elements 206, the replication manager 145 may execute signal conditioning, conduct analog to digital conversion, detection, and synchronization, and execute decoding algorithm, among others.

With the identification of each element 206, the replication manager 145 may replicate, duplicate, or otherwise reproduce the elements 206 as instances 210A-N (hereinafter generally referred to as instances 210) for the respective data stores 170. Each instance 210 may correspond to or include a copy of one or more data elements 206 of the interaction data 204. With the generation of the instances 210, the replication manager 145 can store the instance 210 of the elements 206 onto the respective data stores 170. For each instance 210, the data management service 105 can send, transmit, or otherwise provide the instance 210 to the respective data store 170.

In some embodiments, the replication manager 145 may monitor, maintain, or otherwise track a number of data stores 170 connected to the data management service 105. The number of data stores 170 may increase or decrease according to the entities associated with the clinical trial. For instance, during a first clinical trial, the data management service 105 may connect with 4-8 data stores 170 associated with hospitals, research laboratories, and medical education institutions, among others. Therefore, the replication manager 145 may replicate elements 206 associated with the first clinical trial for each of the 4-8 data stores 170. Continuing on, during a second clinical trial, the data management service 105 may connect with 1-3 data stores 170 associated with hospitals. Therefore, the replication manager 145 may replicate elements 206 associated with the second clinical trial for each of the 1-3 data stores 170. The number of replicated elements 206 corresponds to the number of data stores 170 of entities participating in the clinical trial. For instance, when four data stores 170 (e.g., data store 170A, data store 170B, data store 170C, and data store 170D) are participating in the clinical trial, the replication manager 145 may generate at least four replicated elements 206.

Upon receipt of the instance 210, each data store 170 may store and maintain the data elements 206 of the instance 210. Each data store 170 may store, hold, or otherwise maintain an instance 210 of the elements 206. For instance, the first data store 170A can hold the first instance 210A of the elements 206, whereas the second data store 170B can hold the second instance 210B of the elements 206. The instance 210 may store, hold, or otherwise maintain the elements 206 within a data structure in accordance with the schema of the data store 170. The data structure may be at least one of a dataset, table, array, list, or HashMap, among others, specified by the schema of the data store 170 to store each element 206 to correspond with the user 205 and the clinical trial. For instance, the data structure of the instance 210 may house each element 206 in the plurality of elements 206 in the tables of the data structure. Each instance 210 may represent a duplicate or a copy of the elements 206 across each data store 170. For instance, each instance 210 (instance 210A, instance 210B, instance 210C, etc.) may store the replicated elements 206. Duplicating the elements 206 across each data store 170 may allow the data management service 105 to maintain an instance 210 in each data store 170. Each instance 210 can include the same duplicate elements 206. For example, the data store 170A can include an instance 210A with the elements 206A-D, the data store 170B can include an instance 210B with the elements 206A-D, the data store 170C can include an instance 210C with elements 206A-D, among others. By using the generated data stores 170, the replication manager 145 may generate or replicate elements 206 to increase the number of instances 210 of elements.

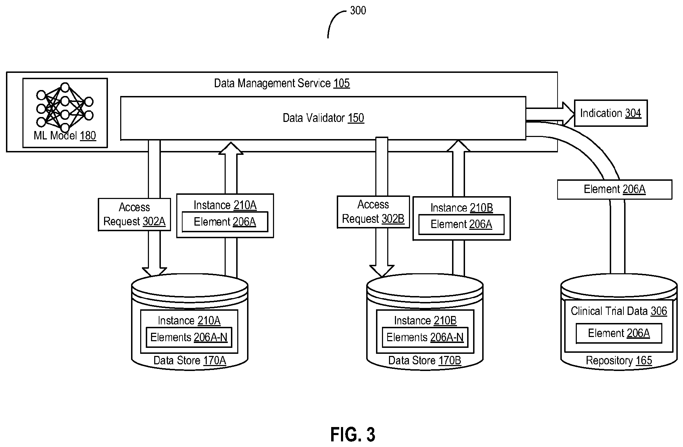

Referring now to FIG. 3, depicted is a block diagram of a process 300 of accessing the plurality of data stores 170 to retrieve an instance 210 of a data element 206 to validate the data element 206 and store the data element 206 in a data repository 165 for the clinical trial data 306. The process 300 may include or correspond to operations performed by the system 100 to reconcile data elements 206 from an interaction with a session. Under the process 300, the data validator 150 may access one or more data stores 170 to retrieve or obtain at least one element 206 for storage in the repository 165. In some embodiments, the data validator 150 may identify or select one or more data stores 170 to retrieve or obtain in accordance with a sequence or schedule. The sequence may specify a priority or order in which the data validator 150 is to access the data stores 170. The sequences may identify a chronological order at which the data store 170 is to be accessed. In the depicted example, the data validator 150 may select or identify the first data store 170A for access.

In some embodiments, the data validator 150 may use the ML model 180 identify or select a data store 170 for accessing the elements 206. The data validator 150 (or the data management service 105) may train the ML model 180 to select or identify data stores 170 from which to access elements 206. The ML model 180 may be trained using a training dataset in accordance with supervised, unsupervised, or weakly supervised learning. The training dataset may identify a number of examples. Each example may include a set of metrics associated with a given data store and a label indication of whether to select the given data store or exclude the given data store from selection. The metrics may be various measures of performance of the data store or any other measure predictive of data integrity at the data store. The metrics may include, for instance, query response time, query throughput, query latency, resource utilization, remaining capacity, index usage, transactions per section, commit rate, rollback rate, failure rate, or downtime, among others. The data validator 150 may apply the set of metrics from each example of the training data to the ML model 180 to generate a selection indication. The selection indication may indicate whether the given data store is to be selected. The data validator 150 may compare the output selection indication with the label indication of the example in the training data to generate a loss metric (e.g., mean square loss, mean absolute error, or cross entropy loss). Using the loss metric, the data validator 150 may update the weights of the ML model 180. This process may be repeated until convergence condition.

Using the ML model 180, the data validator 150 may identify or select a data store 170 for accessing the elements 206. To select, the data validator 150 may determine or identify network metrics for each of the data stores 170. With the identification, the data validator 150 may input or apply the network metrics of a given data store 170 into the ML model 180. Upon applying, the data validator 150 may process the input in accordance with the weights of the ML model 180. From processing, the data validator 150 may produce, output, or otherwise generate a selection indication. When the selection indication identifies that the data store 170 is not to be selected, the data validator 150 may repeat the process by applying the network metrics of another data store 170 to the ML model 180. Conversely, when the selection indication identifies that the data store 170 is to be selected, the data validator 150 may select the data store 170 from which to retrieve the elements 206 (e.g., the first data store 170A as depicted).

The data validator 150 may transmit, send, or otherwise provide the access request 302A to the first data store 170A to access the first data store 170A. The access request 302A may be a signal or communication from the data management service 105 to gain access to the first data store170A, instances 210, or elements 206. For instance, the data management service 105 may transmit the access request 302A to the data store 170 of a hospital to access the instance 210A including element 206A. In another instance, the data validator 150 may transmit the access request 302A to the data store 170 of a research laboratory to access the instance 210A, including element 206A. The access request 302A can include at least one of identity information, clinical trial information, and confirmation, among others, to distinguish between the data stores 170 (e.g., data store 170A, data store 170B). For instance, access request 302A may correspond to data store 170A, access request 302B may correspond to data store 170B, and so on.

The first data store 170A may receive, retrieve, or otherwise obtain the access request 302A from the data validator 150. The data store 170 may extract the information associated with the access request 302A (e.g., identity information, clinical trial information, etc.) to approve or disapprove of the access request 302A based on a match between the information within the access request 302A and the data structure of the instance 210. For instance, the data validator 150 may transmit the access request 302A including the clinical trial and the user 205 associated with the clinical trial to the data store 170. The data store 170 may extract and match the user 205 to the elements 206 within the instance 210A. Once the data store 170 completes the match, the data store 170 can approve the access request 302A.

Upon approval of the access request 302A, the first data store 170A may transmit, send, or otherwise provide the requested instance 210A with the element 206A. In some embodiments, the requested instance 210 can include one or more elements 206. For instance, the requested instance 210A can include elements 206A-C. In another instance, the requested instance 210A can include elements 206A-F. The data store 170A may transmit the instance 210A in accordance with the clinical trial within the access request 302A. From here, the data store 170A may transmit the instance 210A in accordance with the clinical trial.

Once the data store 170A transmits the instance 210 with the element 206A, the data validator 150 may retrieve, receive, or otherwise identify the instance 210A with the element 206A. In some embodiments, the data validator 150 may verify the reception of the instance 210 by transmitting a confirmation request. The confirmation request can include a signature, an indication, and a flag, among others, to acknowledge the safe receipt of the instance 210A with the element 206A. The data management service 105 may allow for communication with clinical trial data 306 stored within the data stores 170 and may interact with a plurality of instances 210 (e.g., instance 210A, instance 210B) to determine which instance to store in the repository 165. Upon receipt, the data validator 150 may parse the instance 210A to retrieve, extract, or otherwise identify the element 206A from the instance 210A.

In addition, the data validator 150 may identify, determine, or otherwise indicate whether the instance 210A of the element 206A is valid or invalid. A valid instance 210A of the element 206A may, for example, have valid values for all the fields in the element 206A. An invalid instance 210A, on the other hand, of the element 206 can include the one or more factors. For instance, the instance 210A of the element 206A may include one or more unexpected values in the interaction data 204. When the user device 120 is offline for long periods of time, the data validator 150 may store and maintain interaction data 204 within the memory of the user device 120.

In some embodiments, to determine whether the instance 210A is valid or invalid, the data validator 150 may generate, calculate, or otherwise determine a confidence value for the instance 210A of the element 206A. The confidence value can indicate, identify, or otherwise specify a degree of validity of the instance 210A of the element 206A. The data validator 150 may determine the confidence value as a function of the values in the element 206A. In general, the data validator 150 may determine the confidence value to be lower, when the element 206 has missing values, is incomplete, or has values outside an expected range for the field, among others. Conversely, the data validator 150 may determine the confidence value to be higher when the element 206 has no missing values, is complete, and has values within the expected range for the field. In some embodiments, the data validator 150 may identify the field type of each field in the element 206. The data validator 150 may compare the value corresponding to the field type with the expected range for the field. The data validator 150 may determine the aggregate confidence value for the element 206 using the confidence value for each field (e.g., as a sum, average, or weighted sum) in the element 206.

In some embodiments, the data validator 150 may use the ML model 180 to identify or determine whether the instance 210A of the element 206A is valid or invalid. In some embodiments, the data validator 150 may use the ML model 180 to calculate, generate, or otherwise determine the confidence value for the instance 210A of the element 206A. The data validator 150 (or the data management service 105) may train the ML model 180 to determine the confidence value. The ML model 180 may be trained using a training dataset in accordance with supervised, unsupervised, or weakly supervised learning. The training dataset may identify a number of examples. Each example may include a sample data element (e.g., with valid, invalid, or null values) and a label indicating whether the sample data element is valid or invalid. The data validator 150 may apply the sample data element from each example to the ML model 180 to produce or generate an output indicating whether the sample element is valid or invalid. The data validator 150 may compare the output indication with the label indication of the example in the training data to generate a loss metric (e.g., a mean square loss, mean absolute error, or cross entropy loss). Using the loss metric, the data validator 150 may update the weights of the ML model 180. This process may be repeated until convergence condition.

Using the ML model 180, the data validator 150 may identify or determine whether the instance 210A of the element 206A is valid or invalid. To determine, the data validator 150 may feed or apply at least a portion of the instance 210A of the element 206A into ML model 180. Upon applying, the data validator 150 may process the input instance 210A of the element 206A in accordance with the weights of the ML model 180. From processing, the data validator 150 may produce, output, or otherwise generate an indication identifying whether the instance 210A of the element 206A is valid or invalid. In some embodiments, the data validator 150 may produce, output, or otherwise generate the confidence value for the instance 210A from the ML model 180. The confidence value may be used to determine whether the instance 210A of the element 206A is valid or invalid as discussed herein below.

The data validator 150 may compare the confidence value with a threshold value to determine whether the data validator 150 can store the element 206A in the repository 165. The threshold value may indicate a maximum degree of validity for the instance 210 of the elements 206 to store the elements 206 within the repository 165. The threshold value may be based on the elements 206 within the repository 165. For instance, the data validator 150 may generate, determine, or otherwise calculate the threshold value, by using the confidence level of the elements 206 within the repository 165 corresponding to the clinical trial. When the confidence value satisfies (e.g., greater than or equal to) the threshold value, the data validator 150 may identify that the instance 210A with the element 206A is valid. Upon the identification of the instance 210A as valid, the data validator 150 may store the element 206A within the repository 165. The data validator 150 may identify the next element 206 (e.g., data element 206B) and repeat the process 300 as detailed herein.

Concurrently with storing the element 206 in the repository 165, the data validator 150 may generate, create, or otherwise produce an indication 304 to indicate that the instance 210A of the element 206A is valid. In some embodiments, the data validator 150 may transmit the indication 304 to an administrative device 110 to identify the stored element 206A. For example, the data validator 150 may transmit the indication 304 of the valid element 206A to the administrative device 110. The administrative device 110 may approve the indication 304 of the stored element 206. In some embodiments, the data validator 150 may detect, identify, or monitor the indication 304 within the administrative device 110. Upon the administrative device 110 approving the indication 304, the data validator 150 may provide the element 206A to each data store 170 to include the element 206A with the highest confidence value. In some embodiments, the data validator 150 may provide the element 206A to each data store 170 to include the element 206A with the highest confidence value without detecting the indication 304.

When the confidence value does not satisfy (e.g., is less than) the threshold value, the data validator 150 may determine or identify that the instance 210A with the element 206A is invalid. With the identification that the element 206A is invalid, the data validator 150 may access the second data store 170B to retrieve, obtain, or otherwise identify a second instance 210B of the corresponding element 206A. To access, the data validator 150 may provide, transmit, or otherwise send an access request 302B to the second data store 170B. Upon approval of the access request 302B, the second data store 170B may transmit, send, or otherwise provide the second instance 210B with the element 206A. The second instance 210B may be different from the first instance 210B. In some instances, the second instance 210B of the element 206A may include one or more factors in comparison to the first instance 210A of the element 206A, thereby, the second instance 210B of the element 206A may include a worse confidence value than the confidence value associated with the first instance 210A of the element 206A. In some instances, the second instance 210B of the element 206A may include one or less factors in comparison to the first instance 210A of the element 206A, thereby, the second instance 210B of the element 206A may include a higher confidence value than the confidence value associated with the first instance 210A of the element 206A.

The data validator 150 may retrieve, receive, or otherwise identify the instance 210B with the element 206A in a similar manner to instance 210A. Upon reception of the instance 210B with the element 206A, the data validator 150 may identify, determine, or otherwise indicate that the instance 210B of the element 206A is valid. To determine that the instance 210B is valid, the data validator 150 may generate, calculate, or otherwise determine the confidence value for the instance 210B of the element 206A. The determination of the confidence value may be in a similar manner as discussed for the instance 210A of the element 206A. With the determination, the data validator 150 may compare the confidence value for the instance 210B of the element 206A with the threshold value. If the confidence value does not satisfy the threshold value, the data validator 150 may determine that the element 206A of the instance 210B is also invalid. Otherwise, if the confidence value satisfies the threshold value, the data validator 150 may determine that the element 206A of the instance 210B is valid.

In some embodiments, when the instance 210A with the element 206A is invalid, the data validator 150 may generate, create, or otherwise produce the indication 304. The indication 304 may identify that the element 206A is invalid across multiple instances 210 or multiple data stores 170. In some embodiments, the data validator 150 may transmit the indication 304 to an administrative device 110 to identify the invalid element 206A. For example, the data validator 150 may transmit the indication 304 of the valid element 206A to the administrative device 110. The administrative device 110 may present the indication 304 to the administrator of the clinical trial. The administrator may use the information provided by the indication 304 to diagnose or modify the digital therapeutic content presented within the session 202, the application 125 itself, or the clinical trial.

In some embodiments, the data validator 150 may compare the confidence value of the instance 210B with the element 206A and the confidence value of the instance 210A with the element 206A, based on a determination that none of the instances 210 of the elements 206 include a confidence value that is above the threshold value. For instance, if each instance 210 with the element 206A on the data stores 170 does not include a confidence value that is higher than the threshold value, the data validator 150 may select the instance 210 with the element 206 with the highest confidence value to store within the repository 165.

Once the instance 210B is valid, the data validator 150 can generate, create, or otherwise produce the indication 304 and transmit the indication to the administrative device 110 as described above. The data validator 150 may store the element 206B onto the repository 165. In some embodiments, the data validator 150 may transmit, send, or provide the element 206B to the repository 165. The repository 165 may store, maintain, or otherwise house the element 206 within the clinical trial data 306. The repository 165 may include elements 206 with the highest confidence value or with confidence values above the threshold value. For instance, the elements 206 within the repository 165 may include confidence values above the threshold value. In this manner, the clinical trial data 306 may include accurate elements 206 for the respective clinical trial. The data management service 105 may utilize the elements 206 within the clinical trial data 306 to provide to a user of the administrative device 110. For instance, the user using the administrative device 110 may analyze the elements 206 of the clinical trial data 306 when evaluating the results of the respective clinical trial.

In some embodiments, to avoid the use of invalid instances 210 with elements 206, the data validator 150 may attempt to access a subsequent data store 170 (e.g., data store 170B) to extract the second instance 210B of the same element 206A. The data validator 150 may identify whether the instance 210B of the element 206A includes a confidence value greater than the threshold value and repeat the process until the instance 210N of the element 206A is greater than the threshold value. In this manner, the system 100 can increase data integrity for the clinical trial. Furthermore, the system 100 can increase the accuracy of the respective clinical trials for each entity associated with the data stores 170.

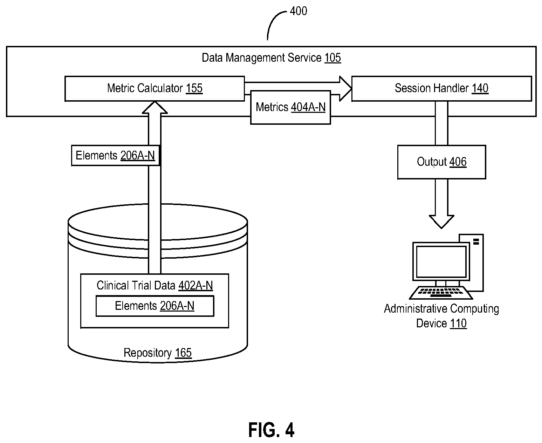

Referring now to FIG. 4, depicted is a block diagram of a process 400 of generating a plurality of metrics 404A-N using the data elements 206 from the data repository 165 and transmitting an output 406 to an administrative device 110. The process 400 may include or correspond to operations performed by the system 100 to generate data elements from an interaction with a session. Under the process 400, the metric calculator 155 executing on the data management service 105 can determine, calculate, or otherwise determine a plurality of metrics 404A-N (hereinafter generally referred to as metrics 404) using the clinical trial data 402 on the repository 165. The repository 165 can store and maintain a clinical trial data 402 herein, including the element 206 identified as valid from the data stores 170 as detailed herein. The metric calculator 155 may access the repository 165 to retrieve one or more of the elements 206. The metric calculator 155 may access the repository 165 to calculate metrics 404, in response to a request or to a trigger associated with the clinical trial. For example, the metric calculator 155 may access the repository 165 to use the element 206 for determining the metrics 404 at set time points during the clinical trial (e.g., 1 week in, 1 month in, or at end of study).

Using the elements 206, the metric calculator 155 may generate the one or more metrics 404 as a function of the elements 206. The metrics 404 may identify or indicate at least one of a degree of efficacy of the application 125, a degree of performance of the application 125, or a degree of severity of a condition to be addressed in the user 205 during the clinical trial, among others. In some embodiments, using the elements 206, the metric calculator 155 may calculate metrics 404 based on the degree of performance of the application 125 while the user 205 is participating in the clinical trial. The degree of performance of the application 125 may indicate a level of functionality (e.g., speed, response time, or error rate) of the application 125 during the clinical trial.

In some embodiments, using the elements 206, the metric calculator 155 may calculate the metrics 404 based on the efficacy of the application 125 to measure a level of deviation from the intended results of the clinical trial. A high efficacy of the application 125 may indicate that the session 202 causes a low level of deviation from the intended results of the clinical trial associated with the respective clinical trial data 402. For instance, the elements 206 may include information as to the number or rate of correct responses to the tasks for addressing mild cognitive impairment (MCI). The metric calculator 155 may determine the degree of efficacy based on the number or rate of correct responses to the task.

In some embodiments, using the elements 206, the metric calculator 155 may calculate the metrics 404 based on a degree of severity of a condition to be addressed in the user 205. The degree of severity may correspond to objective metrics for measuring the effect of the condition on the user 205. For example, for the condition of migraine, the elements 206 may include indications of pain experienced by the user 205 during the clinical trial. The metric calculator 155 may calculate a pain catastrophizing score (PCS) value as one of the metrics 404 using the elements 206. In some embodiments, the metrics 404 may measure a taking of a medication in accordance with the clinical trial, concurrent with the provision of the session 202 or the generation of the data elements 206. For example, the data elements 206 may indicate consumption of medication during the clinical trial, and the metric calculator 155 may determine an indication of whether medication was consumed as one of the metrics 404. With the generation of the metrics 404, the metrics calculator 155 may store and maintain the metrics 404 on the data repository 165 (or another data store 170).

The interface provider 160 can generate, create, or otherwise produce at least one output 406 using the one or more metrics 404. The generation of the output 406 may be based on the digital therapeutic content associated with the user 205, the metrics 404, and the information associated with the metrics 404. For example, the interface provider 160 may select the contents of the output 406 based on a digital therapeutic content associated with the user 205. In some embodiments, the interface provider 160 may generate the output 406 for the session 202 to include the metrics 404 within a summary of a graphical user interface. For instance, the summary within the graphical user interface may include the metrics 404 corresponding to the degree of severity of the condition of the user 205. In some embodiments, the interface provider 160 may generate the output 406 to include an activity log using the information associated with the metrics 404. The interface provider 160 can transmit, send, or otherwise provide the output 406 to the administrative device 110 as a graphical user interface. The information provided in the output 406 may be associated with at least one of the plurality of data stores 170 or the data element 206 on the data repository 165, among others.

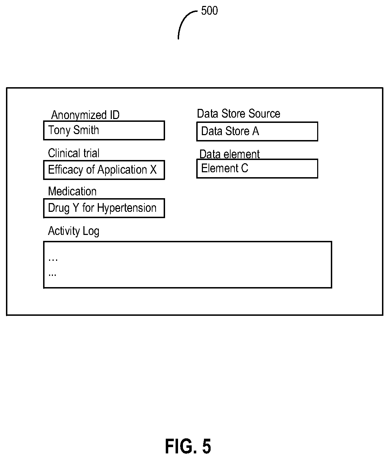

The administrative device 110 can display, generate, or render the output 406 as shown in FIG. 5. FIG. 5 depicts an example of a dashboard 500 provided by the data replication system 100. The dashboard 500 may identify, indicate, or otherwise present information associated with at least one of the plurality of data stores 170 or the element 206 on the data repository 165. For instance, the information associated with the plurality of data stores 170 may indicate the entity associated with the data store 170, the clinical trial the entity is observing, an identifier of the data store 170, and the activities of the participants, among others. The information associated with the element 206 on the repository 165 may include an identifier for the user 205, the clinical trial the user 205 is participating in, the medications taken during the clinical trial, and the instance 210 of the element 206, among others. The user using the administrative device 110 may observe and analyze the information within the dashboard 500 to further the clinical trial.

In this manner, the data management service 105 may allow for a reduction in wasted computing resources when receiving data from user device 120 that transitions from an offline state to an online state. When the user device 120 is offline, the user device 120 may store interaction data 204 within a storage on the user device 120 and allow the user access to the digital therapeutic content and the functionalities of the application 125 online and offline, thereby greatly increasing the overall utility of the user device 120. Once the user device 120 transitions to an online state, the user device 120 may transmit the interaction data 204 to the data management service 105. Using the aspects as described herein, the data management service 105 may reduce the wasted computing resources by copying the interaction data 204 across all data stores 170 associated with the system 100.

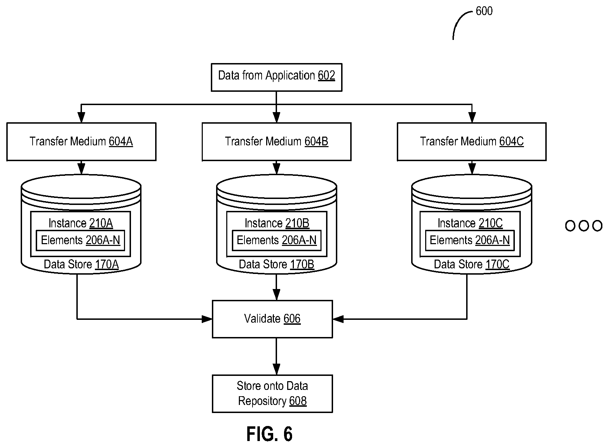

FIG. 6 depicts a block diagram of data replication architecture 600 for clinical data. The data replication architecture 600 may include at least one of the data from the application 602 (e.g., application 125), a plurality of transfer medium 604A-N (generally referred to as transfer medium 604), and the data stores 170. The data stores 170 may validate 606 the instances 210 of the elements 206 and store the elements 206 into the repository 608 (e.g., repository 165). The data from the application 602 may include the interaction data 204, the digital therapeutic content of the session 202, and the elements 206, among others. The data management service 105 may transmit the data from the application 602 to each transfer medium 604. Each transfer medium 604 may be associated with a respective data storage provider to transfer data to the data stores 170.

The data stores 170 may validate 606 (e.g., using the data validator 150) the instances 210 with the elements 206 by matching the elements 206 within the instances 210. In this manner, the validation 606 may identify the optimal instance 210 with the element 206 by comparing each instance 210 with the element 206 to select the instance 210 of the element 206 with the highest confidence value as described above. Once validated 606, the repository 608 may receive the optimal instance 210 with the element 206 and house the element 206. The element 206 may be applied to a calculator (e.g., metric calculator 155) to compute a metric (e.g., metric 404) using at least one data store 170.

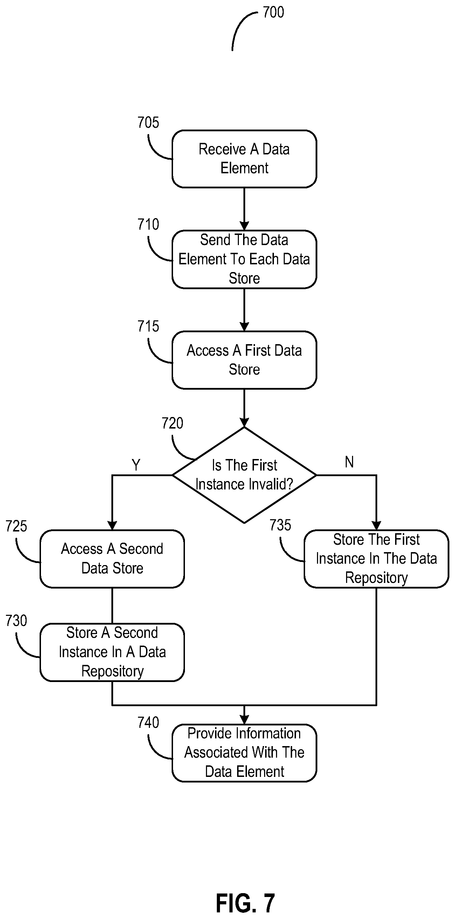

Referring now to FIG. 7, depicted is a flow diagram of a method 700 of maintaining integrity of data during clinical trial for digital therapeutics applications. The method 700 may be implemented or performed using any of the components described herein, such as the data management service 105 and the user device 120, or any combination thereof. Under the method 700, a computing system (e.g., the data management service 105 or the user device 120) may receive a data element generated based on interactions by a user with a digital therapeutic application during a clinical trial, responsive to establishing communications with the digital therapeutic application (705). The computing system may send the data element to each data store of a plurality of data stores (710). The computing system may access a data store of the plurality of data stores to retrieve an instance of the data element (715). The computing system may identify whether a data element in the instance is invalid (720). If the instance of the data element is invalid, the computing system may identify another data store of the plurality of data stores (725) and repeat from (715). If the first instance of the data element is valid, the computing system may store the instance in the data repository (730). The computing system may provide a graphical user interface identifying information associated with at least one of the plurality of data stores or the data element on the data repository (735).

### B. Network and Computing Environment

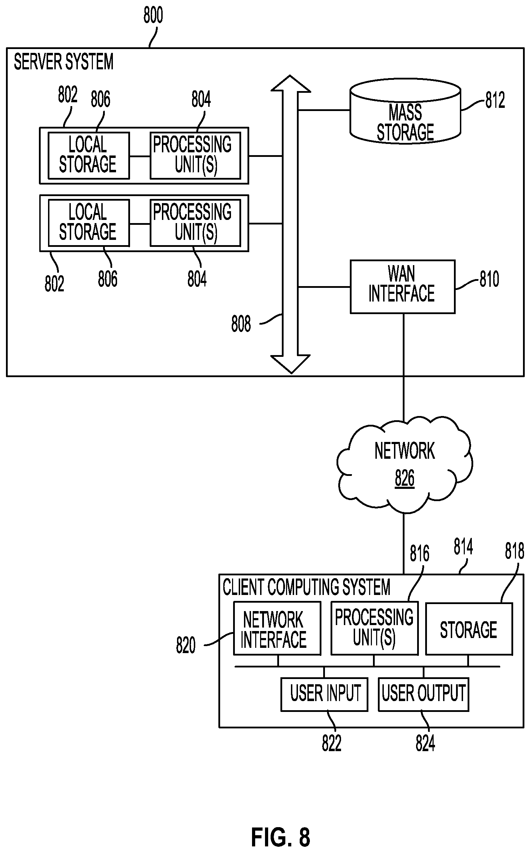

Various operations described herein can be implemented on computer systems. FIG. 8 shows a simplified block diagram of a representative server system 800, client computer system 814, and network 826 usable to implement certain embodiments of the present disclosure. In various embodiments, server system 800 or similar systems can implement services or servers described herein or portions thereof. Client computer system 814 or similar systems can implement clients described herein. The system 100 described herein can be similar to the server system 800. Server system 800 can have a modular design that incorporates a number of modules 802 (e.g., blades in a blade server embodiment); while two modules 802 are shown, any number can be provided. Each module 802 can include processing unit(s) 804 and local storage 806.

Processing unit(s) 804 can include a single processor, which can have one or more cores, or multiple processors. In some embodiments, processing unit(s) 804 can include a general-purpose primary processor as well as one or more special-purpose co-processors, such as graphics processors, digital signal processors, or the like. In some embodiments, some, or all processing units 804 can be implemented using customized circuits, such as application specific integrated circuits (ASICs) or field programmable gate arrays (FPGAs). In some embodiments, such integrated circuits execute instructions that are stored on the circuit itself. In other embodiments, processing unit(s) 804 can execute instructions stored in local storage 806. Any type of processors in any combination can be included in processing unit(s) 804.

Local storage 806 can include volatile storage media (e.g., DRAM, SRAM, SDRAM, or the like) and/or non-volatile storage media (e.g., magnetic or optical disk, flash memory, or the like). Storage media incorporated in local storage 806 can be fixed, removable, or upgradeable as desired. Local storage 806 can be physically or logically divided into various subunits such as a system memory, a read-only memory (ROM), and a permanent storage device. The system memory can be a read-and-write memory device or a volatile read-and-write memory, such as dynamic random-access memory. The system memory can store some or all of the instructions and data that processing unit(s) 804 need at runtime. The ROM can store static data and instructions that are needed by processing unit(s) 804. The permanent storage device can be a non-volatile read-and-write memory device that can store instructions and data even when module 802 is powered down. The term "storage medium" as used herein includes any medium in which data can be stored indefinitely (subject to overwriting, electrical disturbance, power loss, or the like) and does not include carrier waves and transitory electronic signals propagating wirelessly or over wired connections.

In some embodiments, local storage 806 can store one or more software programs to be executed by processing unit(s) 804, such as an operating system and/or programs implementing various server functions, such as functions of the system 100 or any other system described herein, or any other server(s) associated with system 100 or any other system described herein.

"Software" refers generally to sequences of instructions that, when executed by processing unit(s) 804, cause server system 800 (or portions thereof) to perform various operations, thus defining one or more specific machine embodiments that execute and perform the operations of the software programs. The instructions can be stored as firmware residing in read-only memory and/or program code stored in non-volatile storage media that can be read into volatile working memory for execution by processing unit(s) 804. Software can be implemented as a single program or as a collection of separate programs or program modules that interact as desired. From local storage 806 (or non-local storage described below), processing unit(s) 804 can retrieve program instructions to execute and data to process in order to execute various operations described above.

In some server systems 800, multiple modules 802 can be interconnected via a bus or other interconnect 808, forming a local area network that supports communication between modules 802 and other components of server system 800. Interconnect 808 can be implemented using various technologies, including server racks, hubs, routers, etc.

A wide area network (WAN) interface 810 can provide data communication capability between the local area network (e.g., through the interconnect 808) and the network 826, such as the Internet. Other technologies can be used to communicatively couple the server system with the network 826, including wired (e.g., Ethernet, IEEE 802.3 standards) and/or wireless technologies (e.g., Wi-Fi, IEEE 802.11 standards).

In some embodiments, local storage 806 is intended to provide working memory for processing unit(s) 804, providing fast access to programs and/or data to be processed while reducing traffic on interconnect 808. Storage for larger quantities of data can be provided on the local area network by one or more mass storage subsystems 812 that can be connected to interconnect 808. Mass storage subsystem 812 can be based on magnetic, optical, semiconductor, or other data storage media. Direct attached storage, storage area networks, network-attached storage, and the like can be used. Any data stores or other collections of data described herein as being produced, consumed, or maintained by a service or server can be stored in mass storage subsystem 812. In some embodiments, additional data storage resources may be accessible via WAN interface 810 (potentially with increased latency).

Server system 800 can operate in response to requests received via WAN interface 810. For example, one of modules 802 can implement a supervisory function and assign discrete tasks to other modules 802 in response to received requests. Work allocation techniques can be used. As requests are processed, results can be returned to the requester via WAN interface 810. Such operation can generally be automated. Further, in some embodiments, WAN interface 810 can connect multiple server systems 800 to each other, providing scalable systems capable of managing high volumes of activity. Other techniques for managing server systems and server farms (collections of server systems that cooperate) can be used, including dynamic resource allocation and reallocation.

Server system 800 can interact with various user-owned or user-operated devices via a wide-area network such as the Internet. An example of a user-operated device is shown in FIG. 6 as client computing system 814. Client computing system 814 can be implemented, for example, as a consumer device such as a smartphone, other mobile phone, tablet computer, wearable computing device (e.g., smart watch, eyeglasses), desktop computer, laptop computer, and so on.

For example, client computing system 814 can communicate via WAN interface 810. Client computing system 814 can include computer components such as processing unit(s) 816, storage device 818, network interface 820, user input device 822, and user output device 824. Client computing system 814 can be a computing device implemented in a variety of form factors, such as a desktop computer, laptop computer, tablet computer, smartphone, other mobile computing device, wearable computing device, or the like.

Processing unit 816 and storage device 818 can be similar to processing unit(s) 804 and local storage 806 described above. Suitable devices can be selected based on the demands to be placed on client computing system 814. For example, client computing system 814 can be implemented as a "thin" client with limited processing capability or as a high-powered computing device. Client computing system 814 can be provisioned with program code executable by processing unit(s) 816 to enable various interactions with server system 800.

Network interface 820 can provide a connection to the network 826, such as a wide area network (e.g., the Internet) to which WAN interface 810 of server system 800 is also connected. In various embodiments, network interface 820 can include a wired interface (e.g., Ethernet) and/or a wireless interface implementing various RF data communication standards such as Wi-Fi, Bluetooth, or cellular data network standards (e.g., 3G, 4G, LTE, etc.).

User input device 822 can include any device (or devices) via which a user can provide signals to client computing system 814; client computing system 814 can interpret the signals as indicative of particular user requests or information. In various embodiments, user input device 822 can include any or all of a keyboard, touch pad, touch screen, mouse, or other pointing device, scroll wheel, click wheel, dial, button, switch, keypad, microphone, and so on.

User output device 824 can include any device via which client computing system 814 can provide information to a user. For example, user output device 824 can include display-to-display images generated by or delivered to client computing system 814. The display can incorporate various image generation technologies, e.g., a liquid crystal display (LCD), a light-emitting diode (LED) display including organic light-emitting diodes (OLED), a projection system, a cathode ray tube (CRT), or the like, together with supporting electronics (e.g., digital-to-analog or analog-to-digital converters, signal processors, or the like). Some embodiments can include a device such as a touchscreen that functions as both an input and output device. In some embodiments, other user output devices 824 can be provided in addition to or instead of a display. Examples include indicator lights, speakers, tactile "display" devices, printers, and so on.

Some embodiments include electronic components, such as microprocessors, storage, and memory that store computer program instructions in a computer readable storage medium. Many of the features described in this specification can be implemented as processes that are specified as a set of program instructions encoded on a computer readable storage medium. When one or more processing units execute these program instructions, they cause the processing unit(s) to perform various operations indicated in the program instructions. Examples of program instructions or computer code include machine code, such as is produced by a compiler, and files including higher-level code that are executed by a computer, an electronic component, or a microprocessor using an interpreter. Through suitable programming, processing unit(s) 804 and 816 can provide various functionality for server system 800 and client computing system 814, including any of the functionality described herein as being performed by a server or client, or other functionality.

It will be appreciated that server system 800 and client computing system 814 are illustrative and that variations and modifications are possible. Computer systems used in connection with embodiments of the present disclosure can have other capabilities not specifically described here. Further, while server system 800 and client computing system 814 are described with reference to particular blocks, it is to be understood that these blocks are defined for convenience of description and are not intended to imply a particular physical arrangement of component parts. For instance, different blocks can be but need not be located in the same facility, in the same server rack, or on the same motherboard. Further, the blocks need not correspond to physically distinct components. Blocks can be configured to perform various operations, e.g., by programming a processor or providing appropriate control circuitry, and various blocks might or might not be reconfigurable depending on how the initial configuration is obtained. Embodiments of the present disclosure can be realized in a variety of apparatus including electronic devices implemented using any combination of circuitry and software.

While the disclosure has been described with respect to specific embodiments, one skilled in the art will recognize that numerous modifications are possible. Embodiments of the disclosure can be realized using a variety of computer systems and communication technologies, including but not limited to specific examples described herein. Embodiments of the present disclosure can be realized using any combination of dedicated components and/or programmable processors and/or other programmable devices. The various processes described herein can be implemented on the same processor or different processors in any combination. Where components are described as being configured to perform certain operations, such configuration can be accomplished, e.g., by designing electronic circuits to perform the operation, by programming programmable electronic circuits (such as microprocessors) to perform the operation, or any combination thereof. Further, while the embodiments described above may make reference to specific hardware and software components, those skilled in the art will appreciate that different combinations of hardware and/or software components may also be used and that particular operations described as being implemented in hardware might also be implemented in software or vice versa.

Computer programs incorporating various features of the present disclosure may be encoded and stored on various computer readable storage media; suitable media include magnetic disk or tape, optical storage media such as compact disk (CD) or digital versatile disk (DVD), flash memory, and other non-transitory media. Computer readable media encoded with the program code may be packaged with a compatible electronic device, or the program code may be provided separately from electronic devices (e.g., via Internet download or as a separately packaged computer-readable storage medium).

Various aspects of the disclosure will now be set out in the following enumerated clauses in which:
1. A method of maintaining integrity of data during clinical trials of digital therapeutic applications, comprising:
   receiving, by one or more processors, a data element generated based on interactions by a user with a digital therapeutic application during a clinical trial, responsive to establishing communications with the digital therapeutic application;
   sending, by the one or more processors, the data element to each data store of a plurality of data stores;
   accessing, by the one or more processors, a first data store of the plurality of data stores to retrieve a first instance of the data element;
   identifying, by the one or more processors, the first instance of the data element as invalid;
   accessing, by the one or more processors, a second data store of the plurality of data stores to retrieve a second instance of the data element, responsive to the identifying the first instance of the data element in the first data store as invalid; and
   storing, by the one or more processors, the second instance of the data element onto a data repository for the clinical trial.
2. The method of clause 1, further comprising:
   identifying, by the one or more processors, a first instance of a second data element on the first data store as invalid;
   determining, by the one or more processors, that a second instance of the second data element on the second data store is invalid; and
   providing, by the one or more processors, an indication that the second data element on the first data store and the second data store is invalid.
3. The method of clause 1 or clause 2, further comprising:
   identifying, by the one or more processors, the second instance of the data element on the second data store as valid; and
   wherein storing the second data element further comprises storing the second instance of the data element, responsive to identifying the second instance of the data element as valid.
4. The method of any one preceding clause, further comprising:
   identifying, by the one or more processors, a first instance of a second data element on the first data store as valid, and storing, by the one or more processors, the first instance of the second data element from the first data store onto the data repository, without accessing the second data store to retrieve a second instance of the second data element.
5. The method of any one preceding clause, further comprising determining, by the one or more processors, a confidence value indicating a degree of validity of the first instance of the data element, and
   wherein identifying the first instance of the data element as invalid further comprises determining that the confidence value does not satisfy a threshold metric.
6. The method of clause 5, wherein determining the confidence value further comprises applying a machine learning (ML) model to determine the confidence value.
7. The method of any one preceding clause, further comprising determining, by the one or more processors, a plurality of metrics identifying at least one of a degree of efficacy of the digital therapeutic application, a degree of performance of the digital therapeutic application, or a degree of severity of a condition to be addressed in the user during the clinical trial, using the data element stored on the data repository.
8. The method of any one preceding clause, further comprising providing, by the one or more processors, a graphical user interface identifying information associated with at least one of the plurality of data stores or the data element on the data repository.
9. The method of any one preceding clause, further comprising identifying, by the one or more processors, the first data store of the plurality of data stores by applying a machine learning (ML) model.
10. The method of any one preceding clause, wherein receiving the data element further comprises retrieving, from a user device executing the digital therapeutic application, a plurality of data elements generated over a time period during which no communications were established with the user device.
11. The method of any one preceding clause, wherein sending the data element further comprises providing a respective instance of the data element to each data store of the plurality of data stores, the respective instance comprising an encrypted copy of the data element.
12. The method of any one preceding clause, wherein the digital therapeutic application is configured to generate the data element during the clinical trial, in at least partial concurrence with the user being on a medication to address a condition associated with the clinical trial.
13. A system for maintaining integrity of data during clinical trials of digital therapeutic applications, comprising:
   one or more processors coupled with memory, configured to:
   receive a data element generated based on interactions by a user with a digital therapeutic application during a clinical trial, responsive to establishing communications with the digital therapeutic application;
   send the data element to each data store of a plurality of data stores;
   access a first data store of the plurality of data stores to retrieve a first instance of the data element;
   identify the first instance of the data element as invalid;
   access a second data store of the plurality of data stores to retrieve a second instance of the data element, responsive to the identifying the first instance of the data element in the first data store as invalid; and
   store the second instance of the data element onto a data repository for the clinical trial.
14. The system of clause 13, wherein the one or more processors are further configured to:
   identify a first instance of a second data element on the first data store as invalid;
   determine a second instance of the second data element on the second data store is invalid; and
   provide an indication that the second data element on the first data store and the second data store is invalid.
15. The system of clause 13 or clause 14 wherein, the one or more processors are further configured to:
   identify the second instance of the data element on the second data store as valid; and
   wherein, when storing the second data element the one or more processors are further configured to store the second instance of the data element, responsive to identifying the second instance of the data element as valid.
16. The system of any one of clauses 13 to 15, wherein the one or more processors are further configured to:
   identify a first instance of a second data element on the first data store as valid, and
   store the first instance of the second data element from the first data store onto the data repository, without accessing the second data store to retrieve a second instance of the second data element.
17. The system of any one of clauses 13 to 16, wherein the one or more processors are further configured to determine a confidence value indicating a degree of validity of the first instance of the data element, and
   wherein identifying the first instance of the data element as invalid the one or more processors are further configured to determine that the confidence value does not satisfy a threshold metric.
18. The method of clause 17, wherein determining the confidence value further comprises applying a machine learning (ML) model to the first instance to determine the confidence value.
19. The system of any one of preceding clause 13 to 18, wherein the one or more processors are further configured to determine a plurality of metrics identifying at least one of a degree of efficacy of the digital therapeutic application, a degree of performance of the digital therapeutic application, or a degree of severity of a condition to be addressed in the user during the clinical trial, using the data element stored on the data repository.
20. The system of any one of clauses 13 to 19, wherein the one or more processors are further configured to provide a graphical user interface identifying information associated with at least one of the plurality of data stores or the data element on the data repository.
21. The system of any one of clauses 13 to 20, wherein the one or more processors are configured to identify the first data store of the plurality of data stores by applying a machine learning (ML) model.
22. The system of any one of clauses 13 to 21, wherein the one or more processors are further configured to retrieve from a user device executing the digital therapeutic application, a plurality of data elements generated over a time period during which no communications were established with the user device.
23. The system of any one of clauses 13 to 22, wherein the one or more processors are further configured to provide a respective instance of the data element to each data store of the plurality of data stores, the respective instance comprising an encrypted copy of the data element.
24. The system of any one of clauses 13 to 23, wherein the digital therapeutic application is configured to generate the data element during the clinical trial, in at least partial concurrence with the user being on a medication to address a condition associated with the clinical trial.

Thus, although the disclosure has been described with respect to specific embodiments, it will be appreciated that the disclosure is intended to cover all modifications and equivalents within the scope of the following claims.

## Claims

1. A method of maintaining integrity of data during clinical trials of digital therapeutic applications, comprising:
receiving, by one or more processors, a data element generated based on at least one interaction by a user with digital therapeutic application;
sending, by the one or more processors, the data element to each data store of a plurality of data stores;
accessing, by the one or more processors, a first data store of the plurality of data stores to retrieve a first instance of the data element;
accessing, by the one or more processors, a second data store of the plurality of data stores to retrieve a second instance of the data element, responsive to determining the first instance of the data element in the first data store as invalid; and
storing, by the one or more processors, the second instance of the data element onto a data repository.

2. The method of claim 1, further comprising:
determining, by the one or more processors, that a first instance of a second data element on the second data store is invalid; and
providing, by the one or more processors, an indication that the second data element on the first data store and the second data store is invalid.

3. The method of claim 1 or claim 2, further comprising:
wherein storing the second data element further comprises storing the first instance of the data element.

4. The method of any one preceding claim, further comprising:
storing, by the one or more processors, a second instance of the second data element from the first data store onto the data repository, without accessing the second data store to retrieve a third instance of the second data element.

5. The method of any one preceding claim, further comprising determining, by the one or more processors, a confidence value indicating a degree of validity of the first instance of the data element.

6. The method of claim 5, wherein determining the confidence value further comprises applying a machine learning (ML) model to determine the confidence value.

7. The method of any one preceding claim, further comprising determining, by the one or more processors, a plurality of metrics corresponding to at least one of a degree of efficacy of the digital therapeutic application, a degree of performance of the digital therapeutic application, or a degree of severity of a condition to be addressed in the user, using the data element stored on the data repository.

8. The method of any on preceding claim, further comprising providing, by the one or more processors, a graphical user interface comprising information associated with at least one of the plurality of data stores or the data element on the data repository.

9. The method of any one preceding claim, further comprising determining, by the one or more processors, the first data store of the plurality of data stores by applying a machine learning (ML) model.

10. The method of any one preceding claim, wherein receiving the data element further comprises retrieving, from a user device executing the digital therapeutic application, a plurality of data elements generated over a time period during which no communications were established with the user device.

11. The method of any one preceding claim, wherein sending the data element further comprises providing a respective instance of the data element to each data store of the plurality of data stores, the respective instance comprising an encrypted copy of the data element.

12. The method of any one preceding claim, wherein the digital therapeutic application is configured to generate the data element, in at least partial concurrence with the user being on a medication to address a condition.

13. A computer readable storage medium having computer readable instructions stored therein that, when executed by one or more processors, cause the one or more processors to perform the method of any one preceding claim.

14. A system for maintaining integrity of data during clinical trials of digital therapeutic applications, comprising:
one or more processors coupled with memory, configured to perform the method of any one of claims 1 to 12.
